(19) Europäisches Patentamt
European Patent Office
Office européen des brevets

(11) Publication number: 0 144 498 A2

(12) # EUROPEAN PATENT APPLICATION

(21) Application number: 84106231.8

(22) Date of filing: 30.05.84

(51) Int. Cl.$^4$: C 07 C 21/02
C 07 C 17/00

(30) Priority: 12.12.83 US 560139

(43) Date of publication of application: 19.06.85 Bulletin 85/25

(84) Designated Contracting States: BE CH DE FR GB LI NL

(71) Applicant: Union Camp Corporation
1600 Valley Road
Wayne New Jersey 07470(US)

(72) Inventor: McElligott, Lois T.
1158 Wheatsheaf Lane
Abington Pennsylvania 19001(US)

(74) Representative: von Füner, Alexander, Dr. et al,
Patentanwälte Schiff, von Füner Strehl, Schübel-Hopf, Ebbinghaus, Finck Mariahilfplatz 2 u. 3
D-8000 München 90(DE)

(54) **Isomerization of allylic halides with alcohols.**

(57) A method for the isomerization of allylic halides is disclosed, improved by the presence of an alcohol. The improved method of the invention requires less energy for completion of the isomerization and shortens the isomerization times.

EP 0 144 498 A2

Union Camp Corporation

EPAA-31904.0
May 30, 1984

# ISOMERIZATION OF ALLYLIC HALIDES WITH ALCOHOLS

## BACKGROUND OF THE INVENTION

### Field of the Invention

The invention relates to the isomerization of allylic halides.

### Brief Description of the Prior Art

Representative of the prior art is the description given in the U. S. Patent 3,819,730. In spite of the availability of several known methods of isomerizing allylic halides, there has remained a need for economical processes, and processes saving of energy. The method of the present invention is such an improvement over the prior art, requiring relatively low temperatures and short isomerization times and employing a relatively inexpensive catalyst which can be recovered and reused many times.

## SUMMARY OF THE INVENTION

The invention comprises in a method for isomerizing an allylic halide to its allylic isomer which comprises isomerizing the allylic halide in the presence of a

catalytic proportion of a hydrogen halide and a copper catalyst, the improvement which comprises; carrying out the isomerization in the presence of an alcohol.

The term "halide" as used herein is embracive of chloride, bromide and iodide.

## DETAILED DESCRIPTION OF THE PREFERRED EMBODIMENTS OF THE INVENTION

The method of the invention may be employed to isomerize any allylic halide to obtain its allylic isomer. In other words, a tertiary allylic halide may be isomerized to the corresponding secondary or primary allylic halide and the secondary allylic halide may be isomerized to the corresponding primary allylic halide. Representative of an advantageous isomerization according to the method of the invention is the isomerization of linalyl chloride to the geranyl and neryl chlorides according to the schematic formulae:

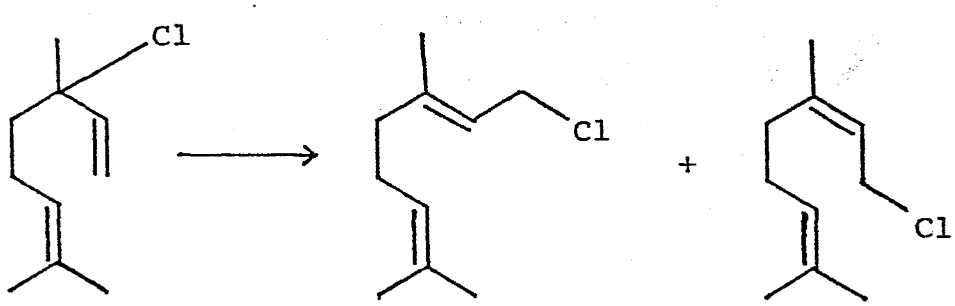

linalyl chloride

geranyl chloride

+

neryl chloride

Especially preferred is chlorine.

It is furthermore preferred to carry out the process at a temperature of 0°C to 50°C.

A preferred copper catalyst is cuprous chloride.

A preferred weight range of the catalytic proportion of copper catalyst is 0.01 to 2.0% of allylic halide and a preferred molar ratio of alcohol to copper catalyst is 0.01 to 5.0.

It is preferred to carry out the isomerization for a period of 0.1 to 10.0 hours.

The preferred alcohol is selected from the group consisting of n-hexanol, cyclohexanol, n-octanol, n-decanol, 3-pentanol, t-butanol and p-dodecylphenol, isopropanol, and isobutanol, and especially from the group of the formula:

$$R — OH$$

wherein R is selected from the group consisting of alkyl having 1 to 25 carbon atoms, inclusive, and aralkyl having 1 to 25 carbon atoms, inclusive.

The isomerization is especially carried out on linalyl chloride.

The method of the invention is not limited to the isomerization of linalyl chloride to geranyl and neryl chlorides but may be used to isomerize any allylic halide to its less-substituted allylic isomer. Other representative allylic halides which may be advantageously isomerized include 3-chloro-3-methyl-1-butene, 3,4-dichloro-1-butene and the like.

The method of the invention is an improvement over isomerizations carried out in the presence of hydrogen halides and copper catalysts alone. Representative of hydrogen halides so employed are hydrogen chloride and hydrogen bromide. In general, catalytic proportions of the hydrogen halide are used. Catalytic proportions are generally within the range of from about 0.01 to 5 percent by weight of the starting halides, preferably 0.1 to 1.0 percent.

The copper catalysts employed may be any copper compound having a valency of 2 or less, including metallic copper. Any copper compound convertible to the halide such as the bromide, iodide or chloride under conditions of the reaction may also be used. Representative of copper catalysts advantageously employed are the chloride, bromide, carbonate, oxide, acetate, formate, sulfate and like derivative cupric and cuprous compounds. Preferred as the copper catalyst in the improved process of the invention is cuprous chloride. Catalytic proportions of the copper catalyst are generally within the weight range of from about

0.01 to 2 percent of the allylic halide starting compound, preferably about 0.5 percent.

A wide range of alcohols may be employed to catalyze the isomerization of allylic halides, according to the method of the invention. Advantageously, the alcohol selected is a fluid under conditions of the process of the invention and is inert to reaction with the isomerization reaction mixture components. Representative of such alcohols are

methanol,
ethanol,
n-hexanol,
n-octanol
n-decanol,
n-tetradecanol,
isopropanol,
isobutanol
3-pentanol,
cyclohexanol,
t-butanol,
2-methyl-2-butanol,
3-ethyl-3-pentanol,
2-ethylhexanol
p-dodecylphenol,

and the like. Preferred alcohols are those of the general formula:

$$R\text{——}OH \qquad (I)$$

wherein R represents alkyl or aralkyl.

The term "alkyl" as used herein means the monovalent moiety obtained upon removal of a hydrogen atom from a parent straight-chain, branched-chain, or cyclic alkane. Representative of alkyl are alkyl of 1 to 25 carbon atoms, inclusive, such as methyl, ethyl, propyl, butyl, pentyl, hexyl, cyclohexyl, heptyl, octyl, nonyl, undecyl, decyl, dodecyl, octadecyl, nonodecyl, eicosyl, heneicosyl, docosyl, tricosyl, tetracosyl, pentacosyl and the isomeric forms thereof.

The term "aralkyl" as used herein means alkyl as defined above wherein a hydrogen atom has been replaced with a monovalent moiety obtained upon removal of a hydrogen atom from an aromatic hydrocarbon. Representative of aralkyl are aralkyl of 7 to 25 carbon atoms, inclusive, such as benzyl, phenethyl, phenpropyl, phenbutyl, phenhexyl, napthoctyl and the like. Preferably, the R moiety will contain from 2 to 22 carbon atoms, inclusive; most preferably 6 to 10 carbon atoms, inclusive.

It will be appreciated that under specific conditions of operating the process of the invention, certain of the above described alcohols of the formula (I) given above have advantages over other compounds of the same general formula.

Selection of a particular alcohol for use under specific process conditions for optimum yields may be made by trial and error techniques.

The alcohol is used in a proportion to isomerize at least some of the allylic halide according to the method of the invention. Such a proportion is generally within the range of from about 0.01 to 10 percent by weight of the halide charge, preferably 0.1 to 5 percent. Optimum proportions will depend to some extent upon the alcohol selected and may be determined by trial and error technique. Generally the preferred molar ratio of alcohol to copper catalyst is from 0.01 to 5.0.

The method of the invention may be carried out by admixing the starting allylic halide with the hydrogen halide, copper catalyst and alcohol in a suitable reaction vessel for a sufficient period of time to effect the desired isomerization. The controlling reaction rate in the method of the invention is the isomerization of the allylic halide to the desired allylic isomer. This is controlled by residence time in the reaction zone. We have found that in isomerization of linalyl chloride the preferred minimum total residence time is within the range of from 0.1 to 10 hours and most preferably 0.5 to 8 hours under preferred operating temperatures.

Although the method of the invention may be carried out under a wide range of operating temperatures, i.e., within the range of from about 0±C. to 50°C., it is preferred to do so at a temperature of from 0°C. to 30°C. and most preferably 0°C. to 20°C.

The method of the invention is not dependent upon pressure, and may be carried out at atmospheric, sub-atmospheric or super-atmospheric pressures.

Progress of the isomerization may be monitored by conventional analytical techniques. When it has been determined that isomerization occurred to a maximum desired point, the product mixture may be passed from the reaction apparatus. The desired allylic halide isomer may then be separated from the reaction mixture employing conventional and known techniques including washing, decantation, distillation and like techniques. The alcohol catalyst may be recovered by conventional procedures, for example by distillation. The recovered alcohol can be reused in the method of the invention.

The following examples describe the manner and process of making and using the invention and set forth the best mode contemplated by the inventors of carrying out the invention but are not to be construed as limiting. All parts given are by weight unless otherwise indicated.

EXAMPLES 1-7

To 8.0g of myrcene hydrochlorides (typically consisting of 51 parts linalyl chloride, 11 parts neryl chloride, 8 parts geranyl chloride, 8 parts alpha-terpinyl chloride and 22 parts hydrocarbons) there is added 0.03g of cuprous chloride, an alcohol compound in an amount indicated in Table 1 below, and 0.02g hydrogen chloride gas. The mixture is stirred at 10°C. for 8 hours. Samples of the reaction mixture are withdrawn at 2 hour intervals, neutralized with aqueous sodium hydroxide and analyzed by gas chromatography. The analytical results are shown in Table 1 below, which also shows the alcohol employed. Linalyl, neryl and geranyl chlorides are abbreviated LCl, NCl, and GCl, respectively.

TABLE 1

| Example No. | Alcohol and Amount used. | Amount of time to reduce LCl content by one-half (hrs.) | GCl:NCl ratio after 8 hours |
|---|---|---|---|
| 1 | none (control) | 5-6 | 1.4 |
| 2 | n-hexanol, 0.03g | $<1$ | 1.5 |
| 3 | cyclohexanol, 0.03g | 1 | 1.5 |
| 4 | n-decanol, 0.04g | 1.5 | 1.5 |
| 5 | 3-pentanol, 0.02g | 1.5 | 1.5 |
| 6 | t-butanol, 0.02g | 2 | 1.4 |
| 7 | p-dodecylphenol, 0.07g | 3.5 | 1.4 |

EXAMPLE 8

To 6.0g of isoprene hydrochlorides consisting of 65 parts 3-chloro-3-methyl-1-butene (abbreviated 3,3,1-CMB) and 35 parts 1-chloro-3-methyl-2-butene (abbreviated 1,3,2-CMB) is added 0.03g of cuprous chloride, 0.05g decanol, and 0.02g hydrogen chloride gas. The mixture is stirred at 10°C for 2 hours. The mixture is neutralized with aqueous sodium hydroxide and analyzed by nuclear magnetic resonance spectroscopy. The analytical results are shown in Table 2 below.

TABLE 2

| Additive | Time Hours | Product Composition (%) 3,3,1-CMB | 1,3,2-CMB |
|---|---|---|---|
| None (control) | 6 | 15 | 85 |
| n-decanol | 2 | 8 | 92 |

WHAT IS CLAIMED:

1. In a method for isomerizing an allylic halide to its allylic isomer which comprises isomerizing the allylic halide in the presence of a catalytic proportion of a hydrogen halide and a copper catalyst, the improvement which comprises carrying out the isomerization in the presence of an alcohol.

2. The process of claim 1 wherein the halogen is chlorine.

3. The process of one of the claims 1 or 2 carried out at a temperature of 0°C to 50°C.

4. The process of one of the preceeding claims wherein the copper catalyst is cuprous chloride.

5. The process of one of the preceeding claims wherein the catalytic proportion of copper catalyst is within the weight range of 0.01 to 2.0% of allylic halide.

6. The process of one of the preceeding claims wherein the molar ratio of alcohol to copper catalyst is 0.01 to 5.0.

7. The process of one of the preceeding claims wherein the isomerization is carried out for a period of 0.1 to 10.0 hours.

8. The process of one of the claims 1 - 7 wherein the alcohol is selected from thė group of the formula:

R —— OH

wherein R is selected from the group consisting of alkyl having 1 to 25 carbon atoms, inclusive, and aralkyl having 1 to 25 carbon atoms, inclusive.

9. The process of claim 8 wherein the alcohol is selected from the group consisting of n-hexanol, cyclohexanol, n-octanol, n-decanol, 3-pentanol, t-butanol an d p-dodecylphenol, isopropanol, and isobutanol.

10. The process of one of the preceeding claims wherein the allylic halide is linalyl chloride.